# EUROPÄISCHE PATENTSCHRIFT

(11) **EP 1 018 499 B1**
(45) Veröffentlichungstag und Bekanntmachung des Hinweises auf die Patenterteilung: **05.02.2003**
(21) Anmeldenummer: 99122829.7
(22) Anmeldetag: 17.11.1999
(51) Int. Cl.: C07C 29/70, C07C 31/12

(54) **Verfahren zur Herstellung von Alkalialkoholaten höherer Alkohole**
Process for the preparation of alkali metal alcoholates of higher alcohols
Procédé de préparation d'alcoolats de métaux alcalins d'alcools supérieurs

(30) Priorität: 05.01.1999 DE 19900073
(43) Veröffentlichungstag der Anmeldung: 12.07.2000
(73) Patentinhaber: Degussa AG, 40474 Düsseldorf (DE)
(72) Erfinder: Hamann, Carl Heinz, Prof. Dr., 26939 Ovelgönne (DE); Helling, Jörg, 64625 Bensheim (DE); Schmittinger, Peter, Dr., 82008 Unterhaching (DE)

(56) Entgegenhaltungen:
- EP-A- 0 810 193
- DE-A- 19 802 013
- US-A- 5 262 133

## Beschreibung

Die Erfindung betrifft ein Verfahren zur Herstellung von Alkalialkoholaten höherer Alkohole aus einem Alkalimetallamalgam und dem freien Alkohol.

Alkalialkoholate sind wichtige Zwischenprodukte u.a. für die Pharmaindustrie, wo sie für galenische Zwecke verwendet werden. Sie werden weiterhin als Katalysatoren bei der Synthese vieler organischer Verbindungen verwendet. Dabei haben vorzugsweise die Alkoholate des Natriums und des Kaliums praktische Bedeutung erlangt. Zur Herstellung von Alkalialkoholaten sind mehrere Methoden bekannt (F.A.Dickes. Ber.Dtsch.Chem.Ges. 63, 2753 [1930]). Lösungen von Alkalihydroxiden in einem Alkohol enthalten das entsprechende Alkalialkoholat im Gleichgewicht. Durch Entfernung des in diesem Gleichgewicht befindlichen Wassers, z.B. durch Destillation, gelangt man zu reinen Alkoholaten. Besonders bei niedrig siedenden Alkoholen wird jedoch für diese Art der Gleichgewichtsverschiebung sehr viel Energie benötigt.

Auf direktem Wege kommt man zu Alkalimetallalkoholaten, indem man ein Alkalimetall in dem entsprechenden Alkohol "auflöst". Dabei reagieren Natrium und Kalium mit niederen Alkoholen, wie Methanol und Ethanol, stürmisch unter Wasserstoffentwicklung. Die träger reagierenden höheren Alkohole, wie Propanole und Butanole, setzt man vorzugsweise oberhalb des Schmelzpunktes des jeweiligen Alkalimetalls um, gegebenenfalls unter Druck und Rühren.

Alkalimetall als Ausgangsstoff für die Alkoholatherstellung ist jedoch teuer. Wirtschaftlicher ist es, als Alkaliquelle die wohlfeilen flüssigen, bei der Chloralkali-Elektrolyse nach dem Quecksilber-Verfahren anfallenden Alkaliamalgame einzusetzen. Auch die Mitverwendung von Katalysatoren zur Beschleunigung der Reaktion von Alkaliamalgam und Alkohol ist bekannt. So wird bei dem Verfahren nach EP-A-0 177 768 eine Schüttung aus stückigem Anthrazit verwendet, deren Oberfläche mit einem Schwermetalloxid oder einer Mischung aus Schwermetalloxiden belegt ist. Alkaliamalgam und Alkohol werden im Gegenstrom kontinuierlich eingespeist und die Alkalialkoholate kontinuierlich abgezogen. Ein Nachteil dieses Verfahrens besteht darin, daß bei der Herstellung von Alkalialkoholaten höherer Alkohole mit akzeptablen Reaktionszeiten nur 60 bis 80 % des mit dem Alkaliamalgam eingetragenen Alkalimetalls umgesetzt werden kann.

Nach dem Vorschlag der deutschen Patentanmeldung 198 02 013.9 läßt sich das im Alkaliamalgam enthaltene Alkalimetall auch mit höheren Alkoholen mit akzeptablen Reaktionszeiten weitergehend umsetzen, wenn man die Reaktion in Gegenwart von Pulverkatalysatoren aus Übergangsmetallcarbiden. -Nitriden oder -Carbonitriden durchführt. Besonders geeignet sind die Metalle Molybdän und Wolfram und von diesen die Carbide. Die Pulverkatalysatoren werden vorteilhaft mit einem mittleren Korndurchmesser von 1 bis 10 µm eingesetzt. Die Umsetzung wird daher als eine mikroheterogen-katalytische Reaktion bezeichnet.

Es wurde nun überraschend gefunden, daß die Reaktionsgeschwindigkeit und damit die Raum-Zeit-Ausbeute bei der Herstellung von Alkalimetallalkoholaten von Alkoholen mit mindestens 3 Kohlenstoffatomen durch Umsetzung eines Alkalimetallamalgams mit dem freien Alkohol in Gegenwart eines Pulverkatalysators aus einem Übergangsmetall-Carbid, -Nitrid oder -Carbonitrid erheblich erhöht wird, wenn man auf das Reaktionsgemisch während der Umsetzung Ultraschall einwirken läßt.

Das neue Verfahren erlaubt die Herstellung der Alkalialkoholate mit einer bis zu 10-fach höheren Reaktionsgeschwindigkeit bzw. Raum-Zeit-Ausbeute, verglichen mit einer Arbeitsweise ohne Ultraschall unter sonst gleichen Bedingungen. Dies ist überraschend, weil langsame Reaktionen (mit reaktionskontrollierter Geschwindigkeit) üblicherweise nicht durch Erhöhung der Durchmischung (d.h. schnelleren Stofftransport zur Katalysatoroberfläche) wesentlich beschleunigt werden können. Infolge der höheren Reaktionsgeschwindigkeit kann man in vorhandenen Apparaten eine erheblich größere Menge an Alkalialkoholaten herstellen oder neue Anlagen für eine gewünschte Kapazität erheblich kleiner auslegen. Diesem Vorteil steht ein vergleichsweise geringer zusätzlicher apparativer und Energieaufwand gegenüber.

Das Verfahren eignet sich besonders für die Umsetzung von Alkoholen mit 3 bis 7 Kohlenstoffatomen und einer primären, sekundären oder tertiären Carbinolgruppe. Überraschenderweise lassen sich auch die notorisch reaktionsträgen Alkoholen mit tertiärer Carbinolgruppe mit guter Reaktionsgeschwindigkeit umsetzen. Alkohole mit mehr als sieben Kohlenstoffatomen lassen sich ebenfalls erfindungsgemäß in ihre Alkalialkoholate überführen, jedoch wird selbst bei erhöhten Temperaturen die Reaktionsgeschwindigkeit mit zunehmender Kohlenstoffzahl zunehmend geringer. Die Alkohole sind vorzugsweise Alkanole, können aber auch olefinische Doppelbindungen oder ein oder zwei Heteroatome, beispielsweise Ethersauerstoffatome, in der Kohlenstoffkette enthalten. Auch Phenol sowie Kresole gelten als höhere Alkohole im Sinne dieser Erfindung. Von den geeigneten höheren Alkoholen seien beispielsweise 1- und 2-Propanol, 1- und 2-Butanol, 2-Methyl-1-propanol (Isobutanol), 2-Methyl-2-propanol (tert.-Butanol), 1-, 2- und 3-Pentanol, 2-Methyl-1-butanol. 2.2-Dimethyl-1-propanol (Neopentylalkohol), 1-, 2- und 3-Hexanol, 2-Propen-1-ol (Allylalkohol), 2-Buten-1-ol (Butenol), 3-Oxa-1-pentanol (Ethylglykol), Phenol sowie o-, m- und p-Kresol genannt. Man wendet den höheren Alkohol zweckmäßig in einem bis zu 20-fachen, insbesondere einem 5 bis 15-fachen stöchiometrischen Überschuß an, bezogen auf das Alkalimetall, und trennt gewünschtenfalls den überschüssigen höheren Alkohol von dem entstandenen Alkalialkoholat ab, z.B. durch Destillation.

Bevorzugte Alkalimetallamalgame sind flüssiges Natrium- und Kaliumamalgam mit Alkalimetallgehalten von 0,1 bis 1, insbesondere von 0.3 bis 0.6 Gewichtsprozent. Solche Alkalimetallamalgame stehen in technischen Mengen aus der Chloralkali-Elektrolyse nach dem Amalgamverfahren zur Verfügung.

Die Mitverwendung eines Übergangsmetall-Carbids, -Nitrids und/oder -Carbonitrids gemäß der deutschen Patentanmeldung 198 02 013.9 ist ein wesentliches Merkmal des erfindungsgemäßen Verfahrens. Ohne den mikroheterogenen Katalysator findet eine Alkoholyse des Alkaliamalgams auch bei Anwendung von Ultraschall praktisch nicht statt. Hinsichtlich der bevorzugten Katalysatoren und der Korngrößen gelten die obigen Angaben. Vorzugsweise beträgt der mittlere Korndurchmesser 1 bis 5 µm, insbesondere 2 bis 3 µm. Man wendet den Katalysator bevorzugt in Mengen von 1 bis 10 Gewichtsprozent an, bezogen auf den höheren Alkohol.

Die Verwendung von Ultraschall zur Förderung der Umsetzung ist ein weiteres wesentliches Merkmal der Erfindung. Hierfür eignet sich ein breites Spektrum von Frequenzen. Vorteilhaft arbeitet man mit Ultraschall von mehr als 16 kHz, insbesondere von 20 bis 40 kHz. Die genannten Frequenzen schließen Bereiche ein, die namentlich für die Ohren junger Menschen noch hörbar sind. Im Sinne dieser Erfindung werden auch diese Bereiche dem Ultraschall zugerechnet. Auch hinsichtlich der Amplitude (oder des spezifischen Energieeintrags) besteht weitgehende Wahlfreiheit. Vorteilhaft beträgt die Amplitude mindestens 0,1 W/cm². Bewährt haben sich z.B. 0.2 bis 20 W/cm². Man setzt übliche Ultraschallgeneratoren ein; wie Ultraschallhörner für direkte oder Ultraschallbäder für indirekte Beschallung.

Man kann das Verfahren nach der Erfindung bei Raumtemperatur (d.h. 20°C) bzw. bei der Temperatur durchführen. die sich infolge der exothermen Reaktion einstellt. Insbesondere bei Alkoholen mit 4 oder mehr Kohlenstoffatomen und sekundärer oder tertiärer Carbinolgruppe kann es im Interesse einer akzeptablen Reaktionsgeschwindigkeit wünschenswert sein, zusätzlich indirekt Wärme zuzuführen. Im allgemeinen arbeitet man bei Temperaturen bis zur Siedetemperatur des jeweiligen Alkohols und bei normalem Druck.

Das erfindungsgemäße Verfahren wird im allgemeinen ohne Mitverwendung eines inerten Löse- oder Verdünnungsmittels durchgeführt. Insbesondere bei Alkoholen mit fünf oder mehr Kohlenstoffatomen, die bei den Reaktionstemperaturen verhältnismäßig viskos sind, kann jedoch der Zusatz eines niedrigviskosen Löse- oder Verdünnungsmittels für die Reaktionsgeschwindigkeit günstig sein. Geeignete inerte Löse- oder Verdünnungsmittel sind, z.B. Ether, wie Diethylether und Tetrahydrofuran.

Man kann das Verfahren nach der Erfindung z.B. diskontinuierlich durchführen indem man das Alkalimetallamalgam mit der Alkohol/Katalysator-Phase überschichtet, durch Rühren für einen guten Kontakt zwischen den Phasen sorgt und Ultraschall auf das Reaktionsgemisch einwirken läßt. Auf das Rühren oder eine andere Form der mechanischen Durchmischung kann man verzichten, wenn man Ultraschall mit hoher Amplitude anwendet. Die Reaktion ist beendet, sobald die Wasserstoffentwicklung zum Erliegen kommt. Die Reaktionszeit beträgt im allgemeinen 2 bis 20 Stunden.

Vorteilhaft führt man das Verfahren jedoch kontinuierlich analog der Zersetzung von Alkalimetallamalgam mit Wasser zur Herstellung von Alkalihydroxiden durch, indem man das Alkaliamalgam und die Alkohol/-Katalysator-Phase im Gleich- oder im Gegenstrom führt.

In beiden Fällen betragen die Umsätze bis zu 100%, bezogen auf das Alkalimetall im Alkaliamalgam. Nach der Phasentrennung kann das Quecksilber in die Chloralkali-Elektrolyse zurückgeführt und aus der Alkohol-Phase, gegebenenfalls nach Abtrennung des Katalysators, das Alkalialkoholat gewonnen werden, zweckmäßig durch Abdestillieren des überschüssigen Alkohols.

Die folgenden Beispiele sollen die Erfindung weiter erläutern, nicht jedoch den Schutzbereich Erfindung begrenzen, wie er sich aus den Patentansprüchen ergibt.

### Beispiele

### Beispiel 1

In einem 2 l-Rundkolben wurden bei 80°C 2.700 g Kaliumamalgam mit einem Gehalt von 15,6 g (0,40 Mol) Kaliummetall mit 390 g (5.26 Mol) tert.-Butanol sowie 30 g (0,15 Mol) Molybdäncarbidpulver mit einem durchschnittlichen Teilchendurchmesser von 2 µm in einem mit Öl gefüllten. beheizten Ultraschallbad gerührt. Der (indirekte) Schalleintrag betrug etwa 0,25 W/cm² bei 35 kHz.

Nach 2,5 Stunden wurde die Reaktion abgebrochen. Die alkoholische Phase wurde vom Amalgam dekantiert und zur Abtrennung von Pulverkatalysator filtriert. Der Alkalirestgehalt im Amalgam (ermittelt durch gasvolumetrische Analyse bei der Zersetzung einer Amalgamprobe mit Schwefelsäure) betrug 5,2 g (0,13 Mol). In der alkoholischen Phase wurden 0,66 g (0,01 Mol) Kaliumhydroxid (bestimmt durch Karl-Fischer-Titration) und 29,72 g (0,26 Mol) Kaliumtert.-butylat (KTB) (bestimmt durch acidimetrische Titration, wobei der KOH-Wert berücksichtigt wird) gefunden. Der Umsatz des Kaliummetalls nach 2,5 h betrug also ca. 61%.

### Beispiel 2

Man arbeitete wie im Beispiel 1 und erhielt aus 2.700 g Kaliumamalgam mit einem absoluten Gehalt an Kaliummetall von 14,85 g (0,38 Mol) und 406 g (5,48 Mol) tert.-Butanol in Gegenwart von 20 g (0,98 Mol) Molybdäncarbid nach 18 h einen quantitativen Umsatz des Kaliummetalls. Neben KTB waren 1.1 g Kaliumhydroxid entstanden.

### Beispiel 3

In einem 1l-Rundkolben wurden bei 80°C 1.300 g Kaliumamalgam mit 9,49 g (0,234 Mol) Kaliummetall und 250 g tert.-Butanol (3,37 Mol) in Gegenwart von 20 g (0,098 Mol) Molybdäncarbid gemäß Beispiel 1 umgesetzt. Der Energieeintrag mittels eines Ultraschallgenerators mit eintauchender Stabsonotrode betrug 15 W/cm² bei 20 kHz. Auf eine mechanische Rührung wurde verzichtet. Nach 5.5 h betrug der Umsatz des im Amalgam enthaltenen Kaliummetalls 85%.

## Patentansprüche

1. Verfahren zur Herstellung von Alkalialkoholaten von Alkoholen mit mindestens 3 Kohlenstoffatomen durch Umsetzung eines Alkalimetallamalgams mit dem freien Alkohol in Gegenwart eines Pulverkatalysators aus einem Übergangsmetall-Carbid. -Nitrid oder -Carbonitrid, **dadurch gekennzeichnet, daß** man auf das Reaktionsgemisch während der Umsetzung Ultraschall einwirken läßt.

2. Verfahren nach Anspruch 1, **dadurch gekennzeichnet, daß** man als höheren Alkohol einen Alkohol mit 3 bis 7 Kohlenstoffatomen und primärer. sekundärer oder tertiärer Carbinolgruppe verwendet.

3. Verfahren nach Anspruch 1, **dadurch gekennzeichnet, daß** der Alkohol ein Alkanol oder ein Alkohol mit einer olefinischen Doppelbindung oder einem oder zwei Heteroatomen in der Kohlenstoffkette ist.

4. Verfahren nach Anspruch 1, **dadurch gekennzeichnet, daß** der Alkohol tert.-Butanol ist.

5. Verfahren nach einem der Ansprüche 1 bis 4, **dadurch gekennzeichnet, daß** man den höheren Alkohol in einem bis zu 20-fachen stöchiometrischen Überschuß, bezogen auf das Alkalimetall im Alkalimetallamalgam, einsetzt.

6. Verfahren nach einem der Ansprüche 1 bis 5, **dadurch gekennzeichnet. daß** als Alkalimetallamalgam ein Natrium- oder Kaliumamalgam mit einem Alkalimetallgehalt von 0,1 bis 1 Gewichtsprozent verwendet wird.

7. Verfahren nach einem der Ansprüche 1 bis 6, **dadurch gekennzeichnet, daß** man mit Ultraschall von mindestens 16 kHz arbeitet.

8. Verfahren nach Anspruch 7, **dadurch gekennzeichnet, daß** der spezifische Energieeintrag mindestens als 0.1 W/cm² beträgt.

9. Verfahren nach einem der Ansprüche 1 bis 8, **dadurch gekennzeichnet, daß** das Quecksilber in die Chloralkali-Elektrolyse zurückgeführt und aus der Alkohol-Phase, gegebenenfalls nach Abtrennung des Katalysators, das Alkalialkoholat gewonnen wird.

## Claims

1. A process for preparing alkali metal alkoxides of alcohols having at least 3 carbon atoms by reacting an alkali metal amalgam with the free alcohol in the presence of a powder catalyst comprising a transition metal carbide, nitride or carbonitride, **characterized in that** ultrasound is allowed to act on the reaction mixture during the reaction.

2. A process according to claim 1, **characterized in that** the higher alcohol used is an alcohol having from 3 to 7 carbon atoms and a primary, secondary or tertiary carbinol group.

3. A process according to claim 1, **characterized in that** the alcohol is an alkanol or an alcohol having an olefinic double bond or one or two heteroatoms in the carbon chain.

4. A process according to claim 1, **characterized in that** the alcohol is tert-butanol.

5. A process according to any one of claims 1 to 4, **characterized in that** the higher alcohol is used in an excess of up to 20 times the stoichiometric amount, based on the alkali metal in the alkali metal amalgam.

6. A process according to any one of claims 1 to 5, **characterized in that** the alkali metal amalgam used is a sodium or potassium amalgam having an alkali metal content of from 0.1 to 1 per cent by weight.

7. A process according to any one of claims 1 to 6, **characterized in that** ultrasound of at least 16 kHz is employed.

8. A process according to claim 7, **characterized in that** the specific energy input is at least 0.1 W/cm².

9. A process according to any one of claims 1 to 8, **characterized in that** the mercury is returned to the chloralkali electrolysis and the alkali metal alkoxide is isolated from the alcohol phase, if appropriate after separating off the catalyst.

## Revendications

1. Procédé de préparation d'alcoolates de métal alcalin d'alcools ayant au moins 3 atomes de carbone, par réaction d'un amalgame de métal alcalin avec l'alcool libre en présence d'un catalyseur en poudre à base de carbure, de nitrure ou de carbonitrure de métal de transition,
**caractérisé en ce qu'**
on fait agir sur le mélange réactionnel pendant la réaction des ultra-sons.

2. Procédé selon la revendication 1,
**caractérisé en ce qu'**
on utilise comme alcool supérieur, un alcool ayant de 3 à 7 atomes de carbone et des groupes carbinol primaires, secondaires ou tertiaires.

3. Procédé selon la revendication 1,
**caractérisé en ce que**
l'alcool est un alcanol ou un alcool ayant une double liaison oléfinique ou bien un ou deux hétéro-atomes dans la chaîne carbonée.

4. Procédé selon la revendication 1,
**caractérisé en ce que**
l'alcool est le butanol tertiaire.

5. Procédé selon l'une des revendications 1 à 4,
**caractérisé en ce qu'**
on met en oeuvre l'alcool supérieur en excès allant jusqu'à 20 fois la stoechiométrie, rapporté au métal alcalin dans l'amalgame de métal alcalin.

6. Procédé selon l'une des revendications 1 à 5,
**caractérisé en ce que**
comme amalgame de métal alcalin, on utilise un amalgame de sodium ou de potassium ayant une teneur en métal alcalin de 0,1 à 1 % en poids.

7. Procédé selon l'une des revendications 1 à 6,
**caractérisé en ce qu'**
on opère avec des ultra-sons d'au moins 16 kHz.

8. Procédé selon la revendication 7,
**caractérisé en ce que**
l'apport spécifique d'énergie s'élève à au moins 0,1 W/cm².

9. Procédé selon l'une des revendications 1 à 8,
**caractérisé en ce que**
le mercure est ramené dans l'électrolyse chloro-alcaline et l'alcoolate de métal alcalin est produit à partir de la phase alcool, le cas échéant après séparation du catalyseur.
